# FASCICULE DE BREVET EUROPEEN

(11) **EP 0 632 732 B1**
(45) Date de publication et mention de la délivrance du brevet: **03.01.1996**
(21) Numéro de dépôt: 93906662.7
(22) Date de dépôt: 24.03.1993
(51) Int. Cl.: A61M 5/142

(54) **POMPE DE PERFUSION DE LIQUIDES MEDICAUX**
MEDIKAMENTENINFUSIONSPUMPE
LIQUID DRUG INFUSION PUMP

(30) Priorité: 24.03.1992 FR 9203775
(43) Date de publication de la demande: 11.01.1995
(73) Titulaire: COMPAGNIE DE DEVELOPPEMENT AGUETTANT, F-69007 Lyon (FR)
(72) Inventeur: WIERNICKI, Michael, V., Broomfield, CO (US); SCATTERGOOD, Mark, G., Broomfield, CO (US)
(74) Mandataire: Maureau, Philippe
(86) Numéro de dépôt international: FR9300296
(87) Numéro de publication internationale: WO9318806

(56) Documents cités:
- DE-A- 3 515 624
- DE-U- 8 901 872
- FR-A- 1 403 779
- US-A- 3 676 026

## Description

La présente invention a pour objet une pompe de perfusion de liquides médicaux.

La perfusion intraveineuse est aujourd'hui une technique essentielle de médication dans le cas de pathologies graves, comme le cancer par exemple.

L'efficacité des traitements est directement liée aux doses prescrites, et limitée par le niveau de toxicité des principes actifs. La précision de l'injection est donc un critère important dans le choix du matériel utilisé. En outre, il a été constaté que l'injection d'un principe actif devait, pour respecter les rythmes biologiques du patient, être effectuée de préférence à certaines heures de la journée, le débit d'injection pouvant être variable tout au cours de la période d'injection pour tenir compte des effets immédiats du principe actif sur l'organisme.

En outre, il a été constaté qu'il était possible, voire avantageux, d'associer plusieurs produits, dont certains peuvent être injectés simultanément à l'aide du même cathéter, et d'autres injectés par des voies différentes.

Une tendance de la médecine actuelle est d'évoluer vers des traitements au domicile du patient, ceci étant plus particulièrement vrai pour les pathologies lourdes telles que traitements du cancer et du S.I.D.A., qui exigent une technicité très importante en milieu hospitalier et un suivi au domicile du patient.

Les matériels de perfusion existant actuellement sur le marché ne tiennent pas compte de cette évolution des soins vers le domicile du patient. Les pompes ambulatoires existantes sont généralement lourdes, encombrantes, et possèdent une autonomie limitée. Les pompes les plus utilisées sont des pompes péristaltiques, qui réalisent l'écrasement d'un tube en polymère ou en silicone pour assurer le déplacement du produit entre un réservoir et le cathéter d'alimentation du patient.

Ce type d'appareil présente l'inconvénient de cumuler des solutions techniques d'encombrement et de poids importants :

L'écrasement du tube exige une force importante qui est obtenue par un système à galets encombrant et lourd. Le moteur doit être dimensionné pour permettre d'obtenir ces forces d'écrasement, ce qui conduit à un poids important. La consommation en énergie du moteur étant importante, son alimentation doit être réalisée par une série de piles ou de batteries volumineuses et lourdes, qui constituent une contradiction avec le principe même du caractère ambulatoire de la pompe.

Une autre solution consiste à utiliser des dispositifs d'aspiration à cylindre et piston, tels que celui illustré par exemple dans DE-A-3 515 624, ou des dispositifs du type pousse-seringue, dans lesquels le piston d'une seringue contenant le produit à injecter est entraîné par un moteur pas à pas. Outre les inconvénients de poids et d'encombrement décrits précédemment, tels systèmes ne permettent pas généralement une programmation, et ne peuvent convenir que pour la perfusion d'un volume limité de liquide.

Le but de l'invention est de fournir une pompe de perfusion de liquides médicaux, à plusieurs canaux, c'est-à-dire susceptible de permettre la perfusion simultanée de plusieurs liquides médicaux, avec possibilité de mélange ou non de ces liquides à l'intérieur de la pompe, qui soit d'un très faible encombrement, et d'un poids réduit, tout en permettant une très grande précision du débit sur une plage de débits très large pouvant varier par exemple entre 1 millilitre par jour et 300 millilitres par heure, et qui offre une sécurité importante pour le patient.

Un autre but de l'invention est de fournir une pompe de perfusion qui comporte une partie assurant le transfert des liquides, qui soit montée de façon amovible sur un boîtier de commande et d'entraînement en mouvements de la pompe.

A cet effet, la pompe qu'elle concerne, du type comprenant au moins un dispositif d'aspiration à cylindre et piston, d'un liquide dans un récipient et de refoulement de celui-ci dans une tubulure reliée au partient, ladite pompe comprenant:
- une première pièce dans laquelle est ménagé au moins un cylindre de dosage qui, contenant un piston animé d'un mouvement alternatif, débouche dans une des faces de la pièce, perpendiculairement à cette face,
- une seconde pièce qui, prenant appui sur la face de la première pièce dans laquelle débouche chaque cylindre, comporte en regard de chaque cylindre, une cavité de diamètre supérieur à celui du cylindre correspondant, et communiquant avec un récipient d'alimentation en liquide,
- une membrane étanche et déformable élastiquement serrée entre les deux pièces, et comportant un orifice en regard de chaque cylindre,
- un disque logé dans chaque cavité de la seconde pièce, dont la face tournée vers la première pièce est étagée et comprend une partie extérieure prenant appui, à travers la membrane sur la face de la première pièce dans laquelle débouche le cylindre correspondant, juste autour de ce cylindre, et une partie centrale faisant saillie du côté de l'intérieur du cylindre, ce disque comportant des trous traversants régulièrement répartis et ménagés à proximité de sa périphérie,
- un ressort exerçant une pression sur le disque en direction de la première pièce, et
- associée à chaque cylindre, une lumière débouchant dans la même face de la première pièce que le cylindre considéré, cette lumière associée à une tubulure d'évacuation ménagée dans cette pièce, étant disposée à proximité du cylindre, en regard de la même cavité que ce cylindre, mais au-delà du disque que contient cette cavité, et étant recouverte par la membrane serrée entre les deux pièces.

La pompe, selon l'invention, est une pompe dans laquelle le dosage de chaque liquide est effectué par un dispositif à cylindre et piston. Il est procédé à l'aspiration du liquide dans le cylindre de dosage, puis au refoulement de celui-ci dans la conduite d'alimentation du cathéter de perfusion.

L'originalité de cette pompe réside essentiellement dans le fait que les deux fonctions de clapets anti-retour, disposés respectivement en amont et en aval du cylindre de dosage, sont remplies par la membrane qui est disposée entre les première et seconde pièces.

Lors d'un mouvement du piston dans un sens d'augmentation du volume du cylindre de dosage, la dépression créée à l'intérieur du cylindre de dosage se traduit par une déformation de la membrane vers le centre du cylindre de dosage, permettant un passage du liquide depuis la cavité ménagée dans la seconde pièce, entre le disque que contient celle-ci et la membrane, puis à l'intérieur de l'orifice central de la membrane vers le cylindre de dosage.

Au contraire, lors d'un mouvement du piston dans un sens de réduction du volume du cylindre de dosage, la pression exercée par le liquide contenu dans ce cylindre sur la membrane, plaque intimement celle-ci contre le disque, tout en permettant une déformation de la partie extérieure de la membrane pour assurer la mise en communication du cylindre avec la lumière adjacente à celui-ci, et reliée à une tubulure d'évacuation du liquide.

Il est possible de ménager, dans les première et seconde pièces, plusieurs pompes de dosage, par exemple quatre pompes, cette réalisation étant très simple dans la mesure où les première et seconde pièces peuvent être constituées par exemple par des plaques parallèlépipèdiques réalisées en une matière synthétique telle qu'un polycarbonate.

Il doit être noté que suivant l'utilisation qui doit être faite des liquides véhiculés par les différents ensembles cylindres-pistons appartenant à une même pompe, il est possible de ménager, à l'intérieur de la première pièce, des réseaux d'évacuation susceptibles ou non de communiquer entre eux. Il suffit pour cela de mouler une première pièce qui est adaptée à la fonction envisagée.

En outre, cette construction en "sandwich" permet d'incorporer, sur les conduits d'évacuation, tous dispositifs de sécurité, tels que dispositifs de détection d'air et dispositifs de surpression. Les tubulures d'évacuation peuvent être ménagées dans la première pièce, de façon à déboucher sur toute leur longueur dans la face de la première pièce en appui contre la seconde pièce, ou au contraire être ménagées dans l'épaisseur de la première pièce, tout en débouchant dans la lumière disposée de façon adjacente à un cylindre de dosage.

Selon une caractéristique de cette pompe, le disque disposé dans la cavité de la seconde pièce comprend une partie cylindrique de grand diamètre, prolongée dans sa partie centrale et du côté du cylindre par une partie cylindrique de diamètre inférieur à celui du cylindre, les trous traversants étant ménagés dans la partie extérieure et débouchant en regard du cylindre.

Cette structure étagée améliore les conditions d'étanchéité, puisque l'appui du disque contre la membrane se fait selon plusieurs arêtes concentriques.

Avantageusement, cette pompe comporte une troisième pièce, en contact avec la première pièce du côté opposé à la seconde pièce, et comportant des alésages prolongeant les cylindres de la première pièce, une membrane d'étanchéité étant montée serrée entre les première et troisième pièces, le piston de chaque pompe comportant deux parties assemblées l'une à l'autre et disposées de part et d'autre de la membrane d'étanchéité, dont celle située dans le cylindre est réalisée en un matériau compatible avec le liquide à véhiculer, et dont l'autre partie est destinée à la fixation des moyens d'entraînement du piston.

Selon un mode de mise en oeuvre, la partie du piston , située du côté de la troisième pièce, est réalisée en un matériau ferro-magnétique, destiné à la fixation avec l'extrémité aimantée d'une tige d'entraînement en mouvement.

Cet agencement de chaque piston en deux parties permet de dissocier les fonctions du piston, en fournissant à la partie située à l'intérieur du cylindre de dosage la fonction d'aspiration et de compression du liquide contenu dans ce cylindre, et en fournissant à la partie située de l'autre côté de la membrane, la seule fonction de fixation des moyens d'entraînement du piston.

Il est ainsi possible d'utiliser, pour la partie du piston située à l'extérieur du cylindre de dosage, un matériau qui n'est pas nécessairement compatible avec le liquide véhiculé, et qui peut être par exemple un matériau ferro-magnétique permettant une fixation immédiate et réversible de cette partie du piston avec l'extrémité aimantée d'une tige d'entraînement en mouvement de ce piston.

Les trois pièces et leurs membranes intermédiaires sont assemblées les unes sur les autres et forment une cassette fonctionnelle indépendante, destinée à être montée de façon amovible sur un boîtier de commande et d'entraînement des pistons.

L'assemblage peut être réalisé par vissage, ou encore par soudage ultra-sons si les trois pièces sont réalisées en matière synthétique. Ces trois pièces forment une cassette fonctionnelle indépendante, qui doit pouvoir être stérilisée, en particulier par des rayons gamma, et qui peut être montée sur un boîtier de commande et d'entraînement des pistons qui contient, pour chaque ensemble de dosage, un moteur, des moyens de transmission du mouvement du moteur au piston, ainsi qu'éventuellement un dispositif de programmation.

Ce boîtier de commande possède une durée de vie importante, tandis que la cassette fonctionnelle n'est utilisée qu'au cours d'un traitement. Lors du changement de traitement, le personnel médical retire la cassette qui vient d'être utilisée, et remplace celle-ci, sur le boîtier de commande, par une nouvelle cassette, adaptée au nouveau traitement.

Il est avantageux de prévoir des éléments de détrompage associés aux moyens d'assemblage entre la cassette et le boîtier, afin d'assurer que le traitement programmé peut bien être mis en oeuvre avec la cassette qui vient d'être montée sur le boîtier.

Selon une autre caractéristique de l'invention, les deux parties du piston possèdent chacune une section qui se rétrécit depuis son extrémité libre vers son extrémité fixée sur l'autre partie.

L'évidement annulaire, ménagé entre les deux parties en vis-à-vis du piston, sert aux mouvements de déformation de la membrane lors du déplacement du piston à l'intérieur de la chambre de dosage.

Dans la mesure où la membrane d'étanchéité est réalisée en un matériau ne possédant pas une excellente souplesse, elle est préformée afin, préalablement à son montage, de présenter au niveau de chaque piston une partie en creux de profil correspondant à celui d'une zone inclinée d'une partie de piston et de la zone d'assemblage des deux parties de piston.

De cette façon, la membrane a tendance à basculer, à l'intérieur du cylindre de dosage, entre deux positions d'équilibre correspondant respectivement à la position reculée et à la position avancée du piston. Il est ainsi possible de imiter sensiblement le volume résiduel à l'intérieur du piston.

De toute façon, l'invention sera bien comprise à l'aide de la description qui suit, en référence au dessin schématique annexé représentant, à titre d'exemples non limitatifs, plusieurs formes d'exécution de cette pompe :
Figure 1 est une vue en perspective d'une pompe destinée à véhiculer quatre liquides distincts ;
Figure 2 est une vue en perspective éclatée des différents composants de la cassette fonctionnelle assurant le transfert du fluide ;
Figures 3 à 6 sont quatre vues en coupe longitudinale de cette cassette au cours de quatre phases de fonctionnement ;
Figures 7 et 8 sont deux vues de dessus de deux variantes de réalisation de la pièce principale et centrale de la cassette.

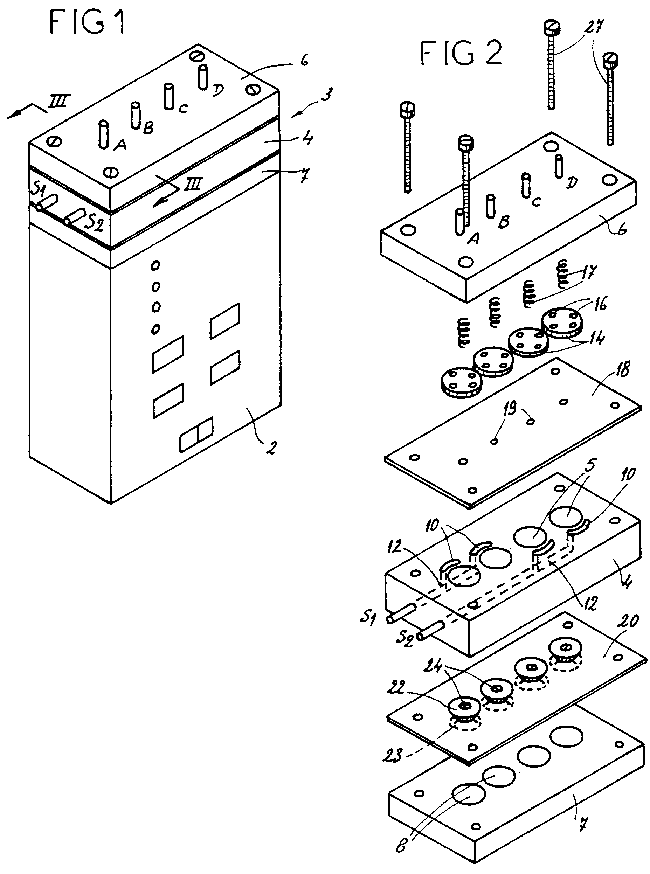

La pompe de perfusion, représentée à la figure 1, est destinée à véhiculer quatre liquides différents. Elle comprend un boîtier **2** contenant des moyens de programmation de l'écoulement de chacun des quatre liquides, et de commande correspondante des pistons des dispositifs de dosage. Ce boîtier contient également des piles électriques pour l'alimentation des moteurs assurant le déplacement des tiges de pistons.

Sur ce boîtier est montée, de façon amovible, une cassette **3** qui correspond à la partie assurant l'aspiration de chaque liquide à l'intérieur d'un récipient constitué par exemple par une poche en matière synthétique, un flacon comportant une prise d'air ou une seringue, et le refoulement de ce liquide dans une tubulure reliée par un cathéter au corps du patient.

Cette cassette est constituée par un empilement de trois pièces parallèlépipèdiques, par exemple en polycarbonate, à savoir une première pièce centrale **4** comportant quatre cylindres de dosage **5**, une seconde pièce **6** équipée de quatre raccords **A**, **B**, **C**, et **D** destinés à la connexion des quatre récipients dans lesquels se trouvent les liquides à transférer, et une troisième pièce **7** dans laquelle sont ménagés des alésages **8** correspondant aux cylindres de dosage **5**, et servant au passage des moyens d'actionnement des pistons, qui seront décrits plus loin, ces moyens d'actionnement étant schématisés au dessin par des tiges **9**.

Dans la première pièce **4** est également ménagée, de façon adjacente à chaque cylindre de dosage **5**, une lumière **10** qui débouche dans la face d'assemblage avec la seconde pièce **6**. Chaque lumière **10** est associée à une tubulure d'évacuation **12**, les tubulures **12** étant, dans la forme d'exécution représentée à la figure 2, regroupées deux par deux, de telle sorte que les cylindres de dosage recevant les liquides des tubulures **A** et **B** comportent des lumières **10** qui sont regroupées au niveau d'une sortie **S1**, alors que les tubulures d'entrées **C** et **D** correspondent à des cylindres de dosage et à des lumières dont les tubulures **12** sont regroupées au niveau d'une sortie **S2**.

Il est également possible de prévoir, dans la pièce **4**, compte tenu de la structure très simple de celle-ci, des dispositifs susceptibles de détecter le passage d'air ou de détecter une surpression.

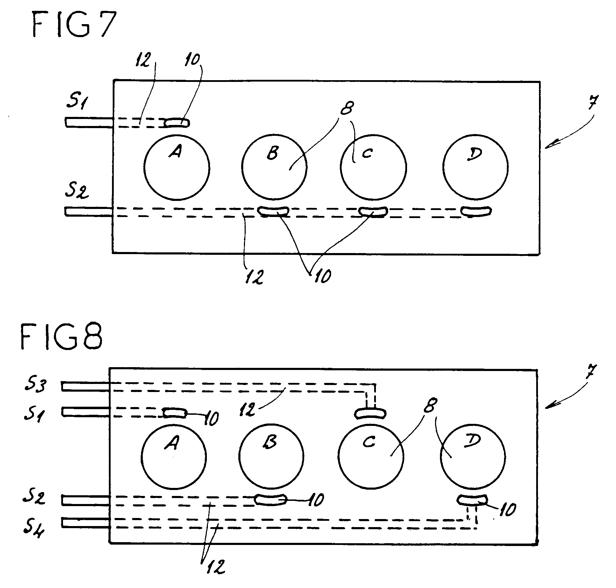

Les figures 7 et 8 représentent des variantes d'exécution de la pièce **4**, dans laquelle les liaisons entre les tubulures de sortie **12** sont différentes.

Dans la forme d'exécution représentée à la figure 7, la tubulure de sortie **S1** correspond seulement à l'entrée **A**, la sortie **S2** résultant du rassemblement des tubulures de sortie correspondant aux liquides amenés par les raccords **B**, **C** et **D**.

Dans la figure 8, chacune des tubulures d'entrée **A**, **B**, **C**, **D** amenant un liquide spécifique, est associée à une tubulure de sortie, respectivement **S1**, **S2**, **S3** et **S4**.

La seconde pièce **6** comporte, en regard de chaque cylindre de dosage **5**, une cavité **13** de diamètre supérieur à celui du cylindre **5** correspondant, et communiquant avec un récipient d'alimentation en liquide par l'une des tubulures, respectivement **A**, **B**, **C** et **D**. Cette cavité sert de logement à un disque **14** dont la face, tournée vers le cylindre de dosage, est étagée. Ce cylindre comprend une partie de grand diamètre, susceptible de prendre appui contre la première pièce **4**, au-delà du cylindre de dosage **5**, prolongée dans sa partie centrale et du côté du cylindre de dosage par une partie cylindrique **15** de diamètre inférieur à celui du cylindre. Des trous traversants **16** dont quatre ont été représentés à la figure 2, sont ménagés dans la partie extérieure du disque **14**, et débouchent en regard du cylindre de dosage **5**.

Le disque **14** est soumis à l'action d'un ressort **17** qui le pousse en regard du cylindre de dosage **5**. L'assemblage des pièces **4** et **6** est réalisé avec interposition d'une membrane **18** déformable, réalisée par exemple en polyéthylène, polypropylène, ou polytétrafluoréthylène. Afin d'éviter une compression excessive de la membrane **18** entre les pièces **4** et **6**, il est prévu, entre celles-ci, des pions formant entretoises d'écartement, non représentés au dessin, qui traversent la membrane **18**. Dans sa partie située en regard du centre de chaque cylindre de dosage, la membrane **18** comporte un orifice **19**.

Le montage entre la première pièce **4** et la troisième pièce **7** est réalisé avec interposition d'une membrane d'étanchéité **20**. Au niveau de chaque cylindre de dosage **5** et de l'alésage **8** correspondant est réalisé le montage d'un piston attelé à la tige **9**. Ce piston est réalisé en deux parties **22** et **23** assemblées l'une à l'autre par une vis **24** par exemple, la partie **22** étant disposée à l'intérieur de la chambre de dosage **5**, et la partie **23** étant disposée, de l'autre côté de la membrane d'étanchéité **20**, à l'intérieur de l'alésage **8**.

La partie de piston **22** est réalisée en un matériau compatible avec le liquide devant traverser la pompe, tandis que la partie de piston **23** peut être réalisée en un matériau non compatible avec un tel liquide, mais possèdant d'autres qualités, telles que celle de permettre un accrochage facile à la tige d'entraînement **9**.

C'est ainsi notamment que la partie **23** de piston peut être réalisée en un matériau ferro-magnétique, et que la tige d'entraînement **9** peut être équipée d'un aimant **25** permettant une fixation et un démontage instantanés de la tige et du piston, lors respectivement de la solidarisation et de la désolidarisation de la cassette **3** et du boîtier **2**.

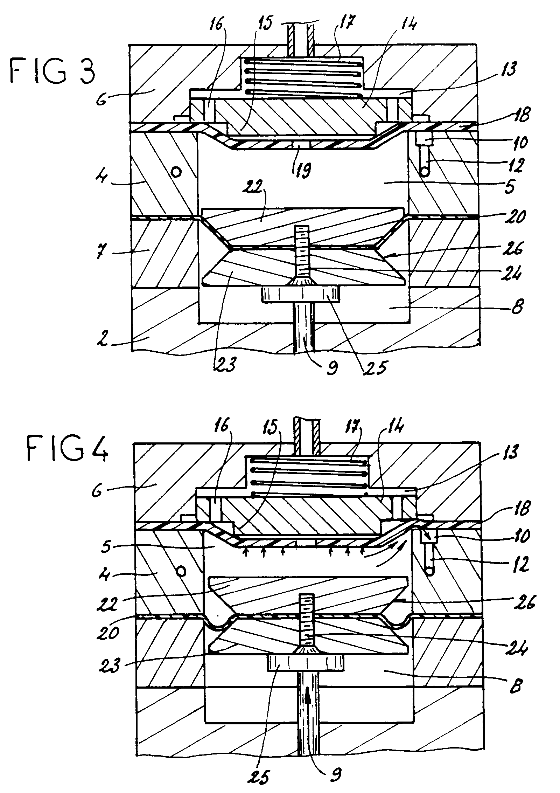

Comme montré notamment aux figures 3 à 6, les deux parties **22** et **23** du piston possèdent chacune une section qui se rétrécit depuis son extrémité libre vers son extrémité fixée sur l'autre partie, ce qui conduit au ménagement d'un évidement annulaire **26**, destiné à faciliter la déformation de la membrane d'étanchéité **20** lors du déplacement du piston.

Dans ce cas encore, la membrane d'étanchéité peut être réalisée en polyéthylène, polypropylène, polytétrafluoréthylène, polyester ou en un tissu renforcé d'élastomère. Dans la mesure où la membrane **20** ne possède pas une parfaite souplesse, il est avantageux de la préformer, en lui donnant une forme correspondant au profil de l'une des zones inclinées du piston, et de la zone d'assemblage entre les deux parties du piston.

L'assemblage entre les différentes pièces **4**, **6**, **7** avec interposition des membranes **18** et **20**, peut être réalisé par exemple par vissage à l'aide de vis **27** dans la forme d'exécution représentée au dessin, étant précisé que la structure en matière synthétique des pièces **4**, **6** et **7** permet leur assemblage par soudage, par exemple par ultra-sons.

Les figures 3 à 6 représentent le mode de fonctionnement de cette pompe, pour un cylindre de dosage.

Dans la forme d'exécution représentée à la figure 3, le piston est en position basse, et le cylindre de dosage **5** est rempli de liquide. Lorsque le piston **22**, **23** est déplacé dans un sens de réduction du volume du cylindre, la pression exercée par le liquide sur la membrane, d'une part, applique intimement celle-ci contre le disque **14**, empêchant tout passage de liquide de la chambre vers l'alimentation et, d'autre part, assure un soulèvement partiel du disque **14** à l'encontre de l'action du ressort **17**, permettant une déformation suffisante de la membrane pour permettre le passage du liquide du cylindre de dosage **5** dans la lumière **10** correspondante, le liquide étant évacué de cette lumière par la tubulure **12**. Ce phénomène est illustré par des flèches à la figure 4.

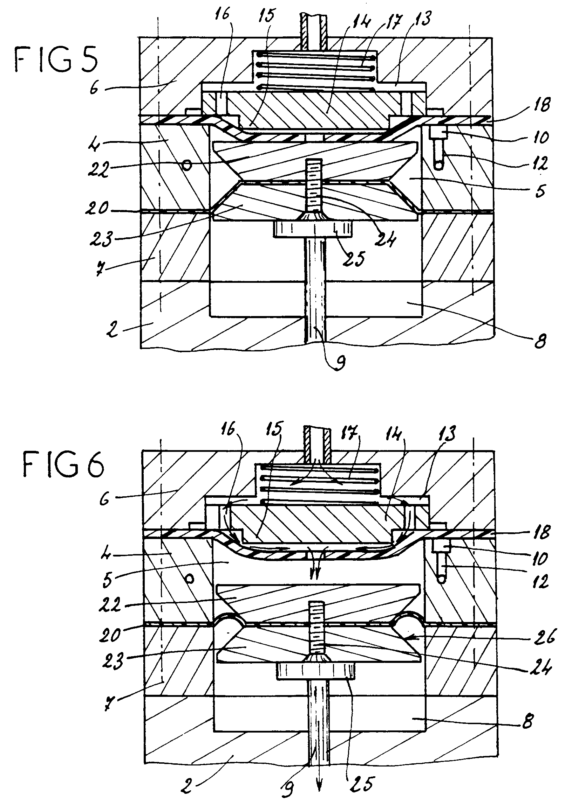

La figure 4 montre également la déformation de la membrane d'étanchéité **20** au cours du déplacement du piston, et illustre bien le fait qu'après une déformation arrondie de la figure 4, la membrane reprend, en fin de course du piston, une position stable, comme montré à la figure 5 qui est l'inverse de la position de départ correspondant à la figure 3.

La figure 5 représente la fin de course haute du piston, dont il est remarquable de constater qu'elle correspond à un volume mort très réduit. Lors de l'entraînement du piston **22**, **23** en sens inverse, une dépression est créée à l'intérieur du cylindre de dosage **5**, qui provoque une déformation de la membrane vers l'intérieur de ce cylindre de dosage, assurant un décollement de la membrane vis-à-vis de la partie centrale **15** du disque **14**, permettant le passage de liquide depuis la cavité **13**, à travers les trous traversants **16**, entre le disque **14** et la membre **18**, puis à travers l'orifice **19** de la membrane **18**, pour réaliser le remplissage du cylindre de dosage **5**. Le mouvement illustré à la figure 6 se poursuit jusqu'à la position de départ qui était illustrée à la figure 3.

Il est intéressant de noter que la membrane **18** joue, vis-à-vis du cylindre de dosage **5** à la fois le rôle de clapet d'entrée et le rôle de clapet de sortie. En outre, en raison de la présence du disque **14**, le dispositif présente toute garantie d'utilisation en cas de surpression à l'entrée, car cette surpression ne peut en aucun cas se traduire par une augmentation de débit susceptible d'être dommageable pour le patient.

Comme il va de soi, l'invention apporte une grande amélioration à la technique existante en fournissant une pompe de perfusion, notamment pour traitement ambulatoire, de structure très simple, d'une grande fiabilité, et susceptible de subir des modifications en vue de son adaptation au type de traitement, en conservant le même boîtier, la même structure pour les pièces **6** et **7** d'extrémité de la cassette en adaptant la pièce centrale **4**, tant en ce qui concerne son épaisseur pour modifier le volume de la chambre de dosage, qu'en ce qui concerne l'aménagement des conduits de sorties en vue de regrouper ou non les sorties des liquides à perfuser.

Comme il va de soi, l'invention ne se limite pas aux seules formes d'exécution de cette pompe, décrites ci-dessus à titre d'exemples ; elle en embrasse, au contraire, toutes les variantes.

C'est ainsi notamment qu'il serait possible de regrouper les pièces **4** et **7** et de ne réaliser un piston, possèdant une parfaite étanchéité par rapport au cylindre, en une seule partie, sans que l'on sorte pour autant du cadre de l'invention.

## Revendications

1. Pompe de perfusion de liquides médicaux, du type comprenant au moins un dispositif d'aspiration à cylindre et piston, d'un liquide dans un récipient et de refoulement de celui-ci dans une tubulure reliée au patient, caractérisée en ce qu'elle comprend :
- une première pièce **(4)** dans laquelle est ménagée au moins un cylindre de dosage **(5)** qui, contenant un piston **(22**, **23)** animé d'un mouvement alternatif, débouche dans une des faces de la pièce, perpendiculairement à cette face,
- une seconde pièce **(6)** qui, prenant appui sur la face de la première pièce **(4)** dans laquelle débouche chaque cylindre **(5)**, comporte, en regard de chaque cylindre, une cavité **(13)** de diamètre supérieur à celui du cylindre correspondant, et communiquant avec un récipient d'alimentation en liquide,
- une membrane **(18)** étanche et déformable élastiquement serrée entre les deux pièces **(4**, **6)**, et comportant un orifice **(19)** en regard de chaque cylindre **(5)**,
- un disque **(14)** logé dans chaque cavité **(13)** de la seconde pièce **(6)**, dont la face tournée vers la première pièce **(4)** est étagée et comprend une partie extérieure prenant appui, à travers la membrane, sur la face de la première pièce dans laquelle débouche le cylindre correspondant, juste autour de ce cylindre, et une partie centrale **(15)** faisant saillie du côté de l'intérieur du cylindre, ce disque comportant des trous traversants **(16)** régulièrement répartis et ménagés à proximité de sa périphérie,
- un ressort **(17)** exerçant une pression sur le disque en direction de la première pièce, et
- associée à chaque cylindre **(5)**, une lumière **(10)** débouchant dans la même face de la première pièce **(4)** que le cylindre **(5)** considéré, cette lumière **(10)** associée à une tubulure **(12)** d'évacuation ménagée dans cette pièce, étant disposée à proximité du cylindre **(5)**, en regard de la même cavité **(13)** que ce cylindre, mais au-delà du disque **(14)** que contient cette cavité, et étant recouverte par la membrane **(18)** serrée entre les deux pièces.

2. Pompe selon la revendication 1, caractérisée en ce que le disque **(14)**, disposé dans la cavité **(13)** de la seconde pièce **(6)** comprend une partie cylindrique de grand diamètre, prolongée dans sa partie centrale et du côté du cylindre de dosage par une partie cylindrique **(15)** de diamètre inférieur à celui du cylindre **(5)**, les trous traversants **(16)** étant ménagés dans la partie extérieure et débouchant en regard du cylindre **(5)**.

3. Pompe selon l'une quelconque des revendications 1 et 2, caractérisée en ce qu'elle comporte une troisième pièce **(7)**, en contact avec la première pièce **(4)** du côté opposé à la seconde pièce **(6)**, et comportant des alésages **(8)** prolongeant les cylindres **(5)** de la première pièce, une membrane d'étanchéité **(20)** étant montée serrée entre les première et troisième pièces **(4**, **7)**, le piston de chaque pompe comportant deux parties **(22**, **23)** assemblées l'une à l'autre et disposées de part et d'autre de la membrane d'étanchéité **(20)**, dont celle **(22)** située dans le cylindre est réalisée en un matériau compatible avec le liquide à véhiculer, et dont l'autre partie **(23)** est destinée à la fixation des moyens d'entraînement **(9)** du piston.

4. Pompe selon la revendication 3, caractérisée en ce que la partie **(23)** au piston située du côté de la troisième pièce **(7)** est réalisée en un matériau ferro-magnétique, destiné à la fixation avec l'extrémité aimantée d'une tige **(9)** d'entraînement en mouvement.

5. Pompe selon l'une quelconque des revendications 3 et 4, caractérisée en ce que les deux parties **(22**, **23)** du piston possèdent chacune une section qui se rétrécit depuis son extrémité libre vers son extrémité fixée sur l'autre partie.

6. Pompe selon la revendication 5, caractérisée en ce que la membrane d'étanchéité **(20)** est préformée afin, préalablement à son montage, de présenter au niveau de chaque piston une partie en creux de profil correspondant à celui d'une zone inclinée d'une partie de piston et de la zone d'assemblage des deux parties de piston.

7. Pompe selon l'une quelconque des revendications 1 à 6, caractérisée en ce qu'elle comporte des ergots ou autres entretoises formant cales d'espacement entre les première et seconde pièces afin de calibrer l'écrasement de la membrane disposée entre elles.

8. Pompe selon l'une quelconque des revendications 3 à 7, caractérisée en ce que les trois pièces **(4**, **6**, **7)** et leurs membranes intermédiaires, sont assemblées les unes sur les autres et forment une cassette fonctionnelle indépendante **(3)**, destinée à être montée de façon amovible sur un boîtier **(2)** de commande et d'entraînement des pistons.

9. Pompe selon la revendication 8, caractérisée en ce qu'elle comporte des éléments de détrompage associés aux moyens d'assemblage du boîtier et la cassette contenant les cylindres.

## Claims

1. Liquid drug infusion pump, of the type comprising at least one device, with cylinder and plunger, for aspirating a liquid from a container and delivering it to a tube connected to the patient, characterized in that it comprises :
- a first member (4) in which is arranged at least one metering cylinder (5) which, containing a plunger (22, 23) actuated by a reciprocating movement, opens into one of the surfaces of the member, perpendicularly to that surface,
- a second member (6) which, supported on the surface of the first member (4) into which each cylinder (5) opens, comprises, opposite each cylinder, a recess (13) whose diameter is greater than that of the corresponding cylinder and which communicates with a liquid supply container,
- a sealed and resiliently deformable membrane (18) sandwiched between the two members (4, 6) and comprising an aperture (19) opposite each cylinder (5),
- a disc (14) housed in each recess (13) of the second member (6), whose surface facing the first member (4) is staggered and comprises an external section supported, via the membrane, on the surface of the first member into which the corresponding cylinder opens, exactly round this cylinder, and a central section (15) projecting on the cylinder interior side, this disc comprising through-holes (16) which are regularly distributed and arranged adjacent to its periphery,
- a spring (17) exerting a pressure on the disc in the direction of the first member, and
- associated with each cylinder (5), a port (10) opening into the same surface of the first member (4) as the cylinder (5) in question, this port (10) being associated with a discharge tube (12) arranged in this member, being arranged adjacent to the cylinder (5), opposite the same recess (13) as this cylinder, but on the other side of the disc (14) which this recess contains, and being covered by the membrane (18) sandwiched between the two members.

2. Pump according to Claim 1, characterized in that the disc (14), arranged in the recess (13) of the second member (6), comprises a cylindrical section of large diameter, prolonged in its central section and on the metering cylinder side by a cylindrical section (15) with a smaller diameter than the cylinder (5), the through-holes (16) being arranged in the external section and opening opposite the cylinder (5).

3. Pump according to one of Claims 1 and 2, characterized in that it comprises a third member (7), in contact with the first member (4) on the side opposite the second member (6) and comprising bores (8) prolonging the cylinders (5) of the first member, a sealing membrane (20) being sandwiched between the first and third members (4, 7), the plunger of each pump comprising two sections (22, 23) assembled one to the other and arranged either side of the sealing membrane (20), the section (22) located in the cylinder being made of a material that is compatible with the liquid to be conveyed and the other section (23) being intended for the fixing of the means (9) by which the plunger is actuated.

4. Pump according to Claim 3, characterized in that the section (23) of the plunger located on the side facing the third member (7) is made of a ferro-magnetic material, intended for fixing to the magnetized end of a movement actuation rod (9).

5. Pump according to one of Claims 3 and 4, characterized in that each of the two sections (22, 23) of the plunger has a section which narrows from its free end towards its end fixed to the other section.

6. Pump according to Claim 5, characterized in that the sealing membrane (20) is pre-formed so that, prior to its assembly, at the level of each plunger it has a hollow-profile section corresponding to that of an inclined zone of a plunger section and of the assembly zone of the two plunger sections.

7. Pump according to one of Claims 1 to 6, characterized in that it comprises lugs or other distance pieces forming spacer wedges between the first and second members in order to gauge the crushing of the membrane arranged between them.

8. Pump according to one of Claims 3 to 7, characterized in that the three members (4, 6, 7) and their intermediate membranes are assembled the ones on the others and form an independent functional case (3) intended to be immovably mounted on a box (2) for controlling and actuating the plungers.

9. Pump according to Claim 8, characterized in that it comprises discriminating elements associated with the means of assembling the box and the case containing the cylinders.

## Patentansprüche

1. Flüssigmedikamenteninfusionspumpe, umfassend wenigstens eine Zylinder- Kolbenvorrichtung zum Ansaugen einer Flüssigkeit in einen Behälter und zum Wiederausstoßen derselben in einen mit dem Patienten verbundenen Rohransatz, dadurch gekennzeichnet, daß sie umfaßt:
- Ein erstes Teil (4), in dem wenigstens ein Dosierzylinder (5) angeordnet ist, der einen durch alternierende Bewegung angeregten Kolben (22, 23) beinhaltet und in eine der Seiten des Teils orthogonal zu dieser Seite einmündet;
- ein zweites Teil (6), das auf der Seite des ersten Teils (4) aufliegt, in welche jeder Zylinder (5) einmündet, das jedem Zylinder (5) gegenüberliegend einen Hohlraum (13) umfaßt, dessen Durchmesser größer ist als jener des entsprechenden Zylinders, und das mit einem Versorgungsbehälter für Flüssigkeit in Verbindung steht;
- eine dichte und elastisch deformierbare Membran (18), welche zwischen den beiden Teilen (4, 6) eingespannt ist und jedem Zylinder (5) gegenüberliegend eine Öffnung (19) umfaßt;
- in jedem Hohlraum (13) des zweiten Teiles (6) aufgenommen eine Scheibe (14), deren zum ersten Teil (4) zugewandte Seite gestuft ist und einen äußeren Abschnitt umfaßt, der über die Membran auf der Seite des ersten Teiles aufliegt, in welche der entsprechende Zylinder einmündet, und zwar gerade um diesen Zylinder herum, und einen zentralen Abschnitt (15) umfaßt, der auf der Seite des Innenraumes des Zylinders vorspringt, wobei diese Scheibe Durchgangslöcher (16) umfaßt, die gleichmäßig verteilt und in der Nähe ihres Umfangs angeordnet sind;
- eine Feder (17), die einen Druck auf die Scheibe in Richtung des ersten Teiles ausübt; und
- jedem Zylinder (5) zugeordnet ein Einlaß (10), der in die gleiche Seite des ersten Teils (4) einmündet wie der betrachtete Zylinder (5), wobei dieser Einlaß (10), einem in diesen Teil ausgebildeten Austragsrohransatz zugeordnet, dem gleichen Hohlraum (13) wie der Zylinder gegenüberliegend in der Nähe dieses Zylinders (5) angeordnet ist, aber jenseits der Scheibe (14), die diesen Hohlraum enthält, und das von der zwischen den beiden Teilen eingespannten Membran (18) bedeckt ist.

2. Pumpe nach Anspruch 1, dadurch gekennzeichnet, daß die Scheibe (14), die in dem Hohlraum (13) des zweiten Teiles (6) angeordnet ist, einen zylindrischen Abschnitt mit großem Durchmesser umfaßt, der in seinem zentralen Abschnitt auf Seiten des Dosierungszylinders durch einen zylindrischen Abschnitt (15) verlängert ist, dessen Durchmesser kleiner ist als jener des Zylinders (5), wobei die Durchgangslöcher (16) in dem äußeren Abschnitt angeordnet sind und in den gegenüberstehenden-Zylinder (5) münden.

3. Pumpe nach einem der Ansprüche 1 und 2, dadurch gekennzeichnet, daß sie ein drittes Teil (7) beinhaltet, das mit dem ersten Teil (4) auf der dem zweiten Teil (6) entgegengesetzten Seite in Kontakt steht und Ausnehmungen (8) umfaßt, die die Zylinder (5) des ersten Teiles verlängern, wobei eine Abdichtungsmembran (20) zwischen dem ersten Teil (4) und dem dritten Teil (7) eingespannt angebracht ist, wobei der Kolben jeder Pumpe zwei Elemente (22, 23) umfaßt, die miteinander verbunden und zu beiden Seiten der Abdichtungsmembran (20) angeordnet sind, wobei jenes im Zylinder Angeordnete (22) aus einem Material gefertigt ist, das sich mit der zu fördernden Flüssigkeit verträgt, und wobei das andere Element (23) zur Befestigung von Antriebsmitteln (9) des Kolbens bestimmt ist.

4. Pumpe nach Anspruch 3, dadurch gekennzeichnet, daß das Kolbenelement (23), das auf Seiten des dritten Teils (7) angeordnet ist, aus einem ferromagnetischen Material hergestellt ist, das zur Befestigung an dem magnetischen Ende eines Bewegungsantriebsschaftes (9) bestimmt ist.

5. Pumpe nach einem der Ansprüche 3 und 4, dadurch gekennzeichnet, daß die beiden Elemente (22, 23) des Kolbens jeweils einen Abschnitt aufweisen, der sich von dessen freiem Ende zu dessen am anderen Element befestigten Ende hin verjüngt.

6. Pumpe nach Anspruch 5, dadurch gekennzeichnet, daß die Abdichtungsmembran (20) vorgeformt ist, um vor ihrer Montage im Bereich jedes Kolbens einen Hohlabschnitt mit einem Profil aufzuweisen, das jenem eines geneigten Bereichs eines Kolbenelementes und des Verbindungsbereichs der beiden Kolbenelemente entspricht.

7. Pumpe nach einem der Ansprüche 1-6, dadurch gekennzeichnet, daß sie Nocken oder andere Zwischenstücke umfaßt, welche Abstandshalter zwischen dem ersten und dem zweiten Teil bilden, um die Druckverformung der zwischen ihnen angeordneten Membran zu kalibrieren.

8. Pumpe nach einem der Ansprüche 3-7, dadurch gekennzeichnet, daß die drei Teile (4, 6, 7) und die zwischen ihnen angeordneten Membranen miteinander zusammengebaut sind und eine abnehmbare Funktionseinheit (3) bilden, die zur abnehmbaren Anbringung auf einem Gehäuse (2) zur Steuerung und zum Antrieb der Kolben bestimmt ist.

9. Pumpe nach Anspruch 8, dadurch gekennzeichnet, daß sie Unverwechselbarkeitselemente umfaßt, die den Montagemitteln des Gehäuses und der die Zylinder beinhaltenden Funktionseinheit zugeordnet sind.
